# EUROPÄISCHE PATENTANMELDUNG

(11) **EP 0 972 536 A1**
(43) Veröffentlichungstag der Anmeldung: **19.01.2000**
(21) Anmeldenummer: 99111390.3
(22) Anmeldetag: 11.06.1999
(51) Int. Cl.: A61M 25/01

(54) **Katheter mit Handgriff**

(30) Priorität: 16.06.1998 DE 19826746
(71) Anmelder: Sulzer Osypka GmbH, 79639 Grenzach-Wyhlen (DE)
(72) Erfinder: Dr.Hebe Joachim, 20149 Hamburg (DE); Gerstmann Hans, 79540 Lörrach (DE)
(74) Vertreter: Schmitt, Hans, Dipl.-Ing.

(57) **Zusammenfassung**

Ein Hilfsgriff (7) kann an einem außerhalb eines Körpers bleibenden Bereich (4) eines Katheters (1) angeklemmt sein oder angeklemmt werden und ist gegenüber dem Katheter (1) in dessen Erstreckungsrichtung verschiebbar, aber auch insbesondere durch die Haltekraft des Benutzers festklemmbar, so daß der Benutzer den Katheter (1) nicht nur mit dessen Handgriff (5), sondern zusätzlich mit diesem Hilfsgriff (7) bewegen, manipulieren oder wenigstens bequem halten kann. Da der Hilfsgriff (7) lösbar befestigt ist, kann er ggfs. auch mehrfach wiederverwendet werden.

## Beschreibung

Die Erfindung betrifft einen Katheter zum Einführen in das Innere des Körpers eines Lebewesens, insbesondere eines Menschen und vorzugsweise in das Gefäßsystem, welcher Katheter einen in Gebrauchsstellung aus dem Körper überstehenden Endbereich mit wenigstens einem Handgriff oder Manipulator oder dergleichen Angreifstelle zur Durchführung von Manipulationen aufweist.

Als "Katheter" ist dabei im vorliegenden Falle jedes flexible, vorzugsweise im Querschnitt zum Beispiel etwa runde oder ovale und/oder schlauchförmige medizinische Behandlungs- und Diagnosehilfsmittel, zum Beispiel auch eine Sonde, gemeint.

Es ist eine Vielzahl unterschiedlicher derartiger Hilfsmittel in unterschiedlichen Formen und für verschiedene Anwendungen bekannt. Beispielsweise kennt man zahlreiche durch Blutgefäße in das Herz einführbare Katheter, um dort Messungen, Stimulationen und/oder Ablationen durchzuführen, wobei auch erforderlich sein kann, die jeweilige Arbeitsspitze eines solchen Katheters von außen her mit drehbaren, schraubbaren oder auch verschiebbaren Handgriffen oder Manipulatoren zu bewegen.

Ein Beispiel für einen derartigen Katheter beschreibt DE 43 20 962 C2. Weitere Beispiele für derartige Katheter mit unterschiedlich gestalteten Handgriffen oder Manipulatoren werden in dieser Druckschrift zitiert, zum Beispiel in EP 0 489 937 A1.

Bei einer Behandlung eines Patienten mit Hilfe eines Katheters wird dieser in der Regel von der behandelnden Person mit beiden Händen erfasst, nämlich einerseits an der als Handgriff ausgebildeten Angreifstelle und andererseits mit der zweiten Hand nahe dem Eintritt dieses Katheters in den Körper. Da solche Katheter einen möglichst geringen Querschnitt haben, um gut eingeführt und betätigt werden zu können, ist vor allem das Halten dieses außerhalb des Körpers befindlichen Bereiches des Katheters nahe seinem Eintritt in den Körper für die zu behandelnde Person mühsam und unbequem. Dies gilt um so mehr, je länger eine derartige Behandlung dauert.

Es besteht deshalb die Aufgabe, einen Katheter der eingangs genannten Art zu schaffen, bei welchem sein Halten am Eintritt in den Körper erleichtert ist und unter Umständen eine zusätzliche Manipulationsmöglichkeit ergibt.

Die Lösung dieser scheinbar widersprüchlichen Aufgabe besteht im wesentlichen darin, daß an dem außerhalb des Körpers bleibenden Endbereich des Katheters ein zusätzlicher relativ zu ihm verschiebbarer, durch eine radiale Druckkraft an der Außenseite des Katheters lösbar festlegbarer Hilfsgriff vorgesehen ist.

Ein solcher Hilfsgriff an dem nicht zum Einführen in den Körper vorgesehenen Endbereich des Katheters ermöglicht es dem Benutzer, also dem behandelnden Arzt, den Hilfsgriff nach dem Einführen des Katheters in eine günstige Lage zu verschieben, in welcher er den Katheter mit seiner zweiten Hand halten oder gegebenenfalls auch bewegen möchte. Dabei ist vorteilhaft, daß dieser Hilfsgriff auf sehr einfache Weise festgelegt werden kann, also der Benutzer auch die eventuelle Verschiebung des Handgriffes relativ zu dem Katheter sehr einfach durchführen kann. Der erfindungsgemäße, in beliebige Position verschiebbare Hilfsgriff eröffnet in vorteilhafter Weise zusätzliche Manipulationsmöglichkeiten an dem Katheter, beispielsweise ein Verdrehen dieses Katheters oder auch eine gewisse axiale Hin- und Herverschiebung in Längserstreckungsrichtung des Katheters. Es könnte also beispielsweise gleichzeitig mit der einen Hand eine Drehbewegung und mit der anderen Hand eine Verschiebebewegung durchgeführt werden, was zum Beispiel beim Positionieren der Katheterspitze im Herzen oder beim Einführen in eine Gefäßabzweigung wünschenswert und zweckmäßig sein kann.

Eine besonders vorteilhafte und zweckmäßige Ausgestaltung der Erfindung, die vor allem auch von der Herstellung und von der Handhabung sowie dem konstruktiven Aufbau des Hilfsgriffes her besonders günstig ist, kann darin bestehen, daß der Hilfsgriff eine den Katheter aufnehmende Innenlängshöhlung aufweist und mindestens bereichsweise derart zusammendrückbar ist, daß er durch Druck auf seine Außenseite lösbar an dem Katheter festklemmbar ist. Somit kann der Benutzer den Hilfsgriff selbst schon dadurch in einer jeweils gewählten Lage festlegen, daß er ihn festhält, was sowieso der Sinn dieses Hilfsgriffes ist. Um den Hilfsgriff zu verschieben, muß die Haltekraft vermindert oder gelockert werden, was jedoch ebenfalls sehr einfach durchführbar ist.

Der Hilfsgriff kann aus weichem Gummi, synthetischem oder Kunstgummi oder Kunststoff bestehen und vom Benutzer durch den Haltedruck mit der Katheteraußenseite verklemmbar sein. Der genannte Werkstoff erlaubt einerseits gut eine Umsetzung der Haltekraft auch in eine Klemmkraft zwischen Hilfsgriff und Katheter und ergibt gleichzeitig einen relativ hohen Reibungskoeffizienten, so daß schon eine relativ geringe Haltekraft eine ausreichende Festlegung des Hilfsgriffes bewirkt.

Eine weitere Ausgestaltung der Erfindung von ganz erheblicher Bedeutung kann darin bestehen, daß der Hilfsgriff - ausgehend von seiner Innenlängshöhlung - wenigstens einen axial und radial verlaufenden Schlitz aufweist, der seine Außen- oder Oberfläche durchsetzt. Dadurch ist es möglich, diesen Hilfsgriff an dem Schlitz aufzuweiten und ihn quer über den Katheter zu bewegen, um ihn an dem Katheter anzubringen oder umgekehrt von dem Katheter wieder zu lösen, ohne daß der Katheter in Längsrichtung in dessen Innenlängshöhlung eingefädelt werden müßte. Somit kann der Hilfsgriff auch auf einen Katheter aufgesetzt werden, der bisher ohne einen solchen Hilfsgriff vorgesehen ist und ferner kann der Hilfsgriff auch mehrfach verwendet werden, selbst wenn der Katheter nur einmal benutzt werden kann oder darf.

Dabei ist es auch möglich, daß der Hilfsgriff durch axiale und radiale Schlitze in wenigstens zwei jeweils einen Anteil der Innenlängshöhlung aufweisende Teile zerlegbar ist und Verbindungsmittel zum Zusammenhalten in Gebrauchsstellung, insbesondere zum gegenseitigen Kuppeln der beiden Teile oder Hälften, aufweist. Dadurch kann das nachträgliche Anbringen an einem Katheter, der eventuell sogar schon verlegt ist, vereinfacht werden, weil einfach die beiden Teile oder Hälften des Hilfsgriffes zueinander passend und dabei den Katheter in sich aufnehmend zusammengefügt werden können.

Eine weitere Ausgestaltung zur Erleichterung des nachträglichen Anbringens oder des Wechselns des Hilfsgriffes kann darin bestehen, daß von der Innenlängshöhlung des Hilfsgriffes aus wenigstens ein weiterer, in radialer und axialer Richtung verlaufender Schlitz oder dergleichen Aussparung vorgesehen ist, welche unterhalb der Oberfläche des Hilfsgriffes endet. Dadurch wird die Aufspreizbarkeit des Hilfsgriffes, insbesondere wenn er nur einen von seiner Innenlängshöhlung radial bis zur Oberfläche reichenden offenen Axialschlitz aufweist, erleichtert, da die Rückstellkraft durch den zusätzlichen, nicht bis zur Oberfläche reichenden Schlitz vermindert wird. Gleichzeitig wird die Nachgiebigkeit auch beim Zusammendrücken größer, so daß relativ geringe Haltekräfte auch zum Festklemmen des Hilfsgriffes mit seiner Innenlängshöhlung an der Katheteraußenseite führen.

Es ist aber auch möglich, daß der Hilfsgriff eine nach seiner Anbringung wirksame Klemme enthält, die durch eine Lösebewegung öffenbar oder lösbar ist. Mit einer solchen in dem Hilfsgriff vorgesehenen Klemme kann er an einer vorgewählten Stelle des Katheters festgelegt werden, so daß der Benutzer den Hilfsgriff auch nicht unabsichtlich eventuell bei Manipulationsbewegungen verschieben kann.

Beispielsweise kann der Hilfsgriff wenigstens eine in radialer Richtung wirksame Druckfeder als Klemme oder Teil einer Klemme enthalten, die in einer klemmenden Druckposition lösbar gehalten und durch eine Ausrastbewegung entspannbar ist. Derartige Druck-Halterungen mit Druckfedern sind beispielsweise aus Druckknopflichtschaltern bekannt, die bei einer ersten Druckbewegung einen Kontakt und entsprechenden Kontaktdruck herstellen und bei einem zweiten Drücken wieder in entspannte Lage zurückspringen.

Eine andere oder zusätzliche Möglichkeit besteht darin, daß als Klemme eine exzentrisch angeordnete Rolle innerhalb des Handgriffes gelagert ist, deren Achse quer zu der Längsmittelhöhlung des Hilfsgriffes orientiert ist und die zum Lösen entgegen ihrer Klemmstellung verdrehbar ist. Aufgrund der Exzentrizität führt eine Verdrehung in der einen Richtung zu einer immer größeren Annäherung an die Innenlängshöhlung bzw. einen Eingriff in diese, so daß ein dort verlaufender Katheter verklemmt wird. Eine entgegengesetzte Verdrehung entfernt den Bereich größerer oder großer Exzentrizität wieder von dieser Innenlängshöhlung, so daß sie und der in ihr verlaufende Katheter freigegeben werden und dann der Hilfsgriff in Längsrichtung verschoben werden kann.

Der die Oberfläche des Hilfsgriffs durchsetzende Schlitz kann an zumindest einem Ende des Hilfsgriffs eine insbesondere etwas trichterförmige Erweiterung haben. Dadurch kann das seitliche Aufstecken des Hilfsgriffes auf den Katheter an einer für den Benutzer günstigen Stelle erleichtert werden, also ein Einfädeln des Katheters in die Innenlängshöhlung vermieden werden.

Eine weitere Ausgestaltung der Erfindung und insbesondere des Gedankens, daß der Hilfsgriff eine nach seiner Anbringung wirksame Klemme enthalten kann, kann darin bestehen, daß die Innenlängshöhlung und/oder der Hilfsgriff selbst als verformbare oder lösbare Klemme ausgebildet sind. In einem solchen Fall enthält der Hilfsgriff also die Klemme durch eine entsprechende Ausbildung, was einfacher und preiswerter herstellbar ist, als eine eigenständige Klemme oder Klemmvorrichtung in den Hilfsgriff einzubauen.

Ausgestaltungen dieses Gedankens, den Hilfsgriff eine Klemme enthalten zu lassen, die durch die Ausbildung der Innenlängshöhlung und/oder des Hilfsgriffes selbst gebildet wird, sind Gegenstand der Ansprüche 12 bis 14. Vor allem bei Kombination der Maßnahme nach Anspruch 12 und 13 ergibt sich ein Hilfsgriff mit Innenlängshöhlung, die einen länglichen, eventuell ebenfalls etwa schlitzförmigen Querschnitt hat und ein Katheter durch die einander naheliegenden Wandungsbereiche dieser Innenlängshöhlung selbsttätig klemmend erfaßt wird. Wird rechtwinklig zu dieser Verklemmung, also in Richtung der längeren Abmessung der Innenlängshöhlung von außen her ein Druck ausgeübt, wird bekanntermaßen ein solches elastisches Teil verformt, so daß die nah beieinanderliegenden Wandungsbereiche jeweils in dem Sinne zurückweichen, daß die längere Abmessung unter dieser Druckwirkung kürzer und gleichzeitig die geringere Abmessung breiter wird, so daß dann der eingeklemmte Katheter freigegeben wird. Wird die Druckkraft, die der Benutzer zweckmäßigerweise mit einer Hand aufbringt, wieder reduziert oder aufgehoben, klemmt sich der Hilfsgriff aufgrund seiner Formgebung wieder selbsttätig an dem Katheter fest.

Die Maßnahme des Anspruches 14 bewirkt dabei, daß durch den Druck in Richtung der größeren Abmessung des Querschnittes der Innenlängshöhlung der zum seitlichen Einführen des Katheters dienende Schlitz gleichzeitig fester verschlossen wird, so daß ein ungewolltes Austreten des Katheters aus diesem Schlitz vermieden werden kann.

Durch die Maßnahme des Anspruches 15 wird dem Benutzer die Ausübung einer Druckkraft in der richtigen Richtung erleichtert, sei es bei einem Hilfsgriff, der durch die Druckkraft in der jeweiligen Position fixiert werden soll, sei es bei einem Hilfsgriff, bei welchem die zeitweilige Positionierung durch dessen Eigenspannung erfolgt und die Druckkraft zum Lösen der Klemmwirkung bei einer Verschiebung oder Lösung des Hilfsgriffes aufgebracht werden soll.

Bei dem Arbeiten mit derartigen Kathetern und dem Hilfsgriff kann die Verbindungsstelle zwischen Katheter und Hilfsgriff trocken oder auch feucht sein. Dies kann die zeitweilige Festlegung des Hilfsgriffes oder auch seine Verschiebbarkeit beeinflussen. Um sowohl bei einem trockenen als vor allem auch einem nassen Katheter mit einer möglichst geringen Druckkraft zum Festlegen des Hilfsgriffes auskommen zu können, was auch bei einem solchen Hilfsgriff vorteilhaft sein kann, bei dem die Festlegung durch eine Ausbildung des Hilfsgriffes als lösbare Klemme vorgesehen ist, ist es zweckmäßig, wenn der Hilfsgriff in seiner Innenlängshöhlung zumindest bereichsweise mit einer unter Druck gleithemmenden oder rutschfesten Auskleidung versehen ist. Es sind zum Beispiel Folien bekannt, die einerseits eine glatte Oberfläche haben, andererseits aber schon unter geringem Druck an der Gegenfläche haften und praktisch nicht mehr verschiebbar sind. Wird die Innenlängshöhlung mit einer solchen Auskleidung versehen, genügt also sowohl an einem trockenen als auch an einem feuchten Katheter eine geringe Druckkraft, um den Hilfsgriff jeweils in einer gewünschten Position festzulegen und ihn dann auch betätigen zu können.

Die Auskleidung kann eine Folie mit an ihrer Oberfläche überstehenden Partikeln, zum Beispiel eine Schmirgelfolie, oder eine Folie mit glatter Oberfläche und hohem Reibungskoeffizienten, insbesondere aus Polyurethan, sein. Im ersteren Falle wird durch die überstehenden Partikel an der Oberfläche des Katheters ein zeitweiliger Formschluß erzielt, während im zweiten Fall der hohe Reibkoeffizient der entsprechenden Folie ausgenutzt wird, ohne daß die Außenseite des Katheters mit Eindrücken versehen werden muß.

Eine weitere Ausgestaltung des Hilfsgriffes für eine praktische Betätigung und insbesondere auch für die Möglichkeit, durch eine einmalige Manipulation den Hilfsgriff zeitweilig festzulegen, ihn aber auch wieder leicht lösen zu können, kann es zweckmäßig sein, wenn die lichte Weite des Innenquerschnittes der Innenlängshöhlung oder der Auskleidung zumindest bereichsweise durch Längszug und/oder durch Verdrillen verringerbar und in so verformter Position lösbar festlegbar ist. Es ist bekannt, daß bei einer elastischen Längsausdehnung oder einer Verdrillung eines schlauchartigen Gebildes dessen Innenquerschnitt vermindert wird. Diese Erkenntnis kann also zum zeitweiligen Festklemmen des Hilfsgriffes ausgenutzt werden.

Besonders vorteilhaft kann es dabei sein, wenn die Auskleidung der Innenlängshöhlung in einem Endbereich drehfest mit dem Hilfsgriff und an dem anderen Endbereich verdrehbar und lösbar festlegbar ausgebildet ist und vorzugsweise einen gegenüber dem Hilfsgriff axial vorstehenden Kopf zum Verrasten gegenüber dem Hilfsgriff in unterschiedlichen Drehpositionen hat, wobei die Auskleidung und/oder der Hilfsgriff in axialer Richtung elastisch sind. Bei einer derartigen Ausführungsform kann der Benutzer also den mit der Auskleidung verbundenen und über den Hilfsgriff überstehenden Kopf erfassen und verdrehen, wodurch die zeitweilige Verklemmung mit dem Katheter erfolgt. Danach braucht also der Benutzer dann keine weitere Kraft aufzubringen, um den Hilfsgriff in der einmal gewählten Position am Katheter zu fixieren. Dies kann bei einer Operation oder Manipulation mit einem Katheter von erheblichem Vorteil sein. Soll der Hilfsgriff wieder gelöst und/oder in eine andere Position gebracht werden, genügt es, die Verrastung des vorstehenden Kopfes zu lösen und die Verdrillung oder Verdrehung der Auskleidung der Innenlängshöhlung wieder rückgängig zu machen, um den Hilfsgriff gegenüber dem Katheter so weit zu lockern, daß er wieder bequem verschoben werden kann.

Es besteht also die Möglichkeit, den zusätzlichen Hilfsgriff sehr einfach aufzubauen und mit einem Längsschlitz zu versehen, um ihn auch schnell montieren zu können, wobei lediglich die Haltekraft des Benutzers den Hilfsgriff auch an einer beliebig gewählten Stelle des Katheters festlegt und diese Stelle auch je nach Erfordernis beim Manipulieren schnell geändert werden kann. Die andere Möglichkeit besteht darin, den Hilfsgriff mittels einer Klemme zu fixieren, die auf einfache Weise gelöst werden kann, so daß der Hilfsgriff auch in einer solchen Ausführungsform in eine jeweils günstige Position gebracht werden kann, wobei zwar dann ein höherer Aufwand wegen der vorzusehenden Klemme anfällt, der Benutzer aber den Hilfsgriff so fixieren kann, daß auch größere Kräfte, beispielsweise auch Zugkräfte aufgebracht werden können, ohne daß die Gefahr eines ungewollten Verschiebens des Hilfsgriffes besteht.

Dabei kann die erwähnte Klemme auch durch die Gestaltung des Hilfsgriffes selbst an diesem oder seiner Innenlängshöhlung oder einer Auskleidung der Innenlängshöhlung ausgebildet sein.

Nachstehend sind Ausführungsbeispiele der Erfindung anhand der Zeichnung näher beschrieben. Es zeigt in zum Teil schematisierter Darstellung:
- Fig.1: eine Ansicht eines in seinem Verlauf unterbrochen dargestellten Katheters, der zum Manipulieren seiner Arbeitsspitze einen Handgriff und erfindungsgemäß außerdem mit geringem Abstand zu diesem Handgriff in einem außerhalb des Körpers bleibenden Endbereich einen Hilfsgriff aufweist,
- Fig.2: eine Ansicht des außerhalb des Körpers bleibenden Teiles eines Katheters und eine Einführvorrichtung, durch welche der Katheter in einen Körper eintritt, wobei nahe dieser Eintrittsstelle der Hilfsgriff angeordnet ist und gleichzeitig angedeutet ist, wie ein Benutzer einerseits mit der rechten Hand den dem Katheter zugehörenden Handgriff und andererseits den erfindungsgemäß vorgesehenen Hilfsgriff erfaßt,
- Fig.3: eine Stirnansicht,
- Fig.4: einen Längsschnitt und
- Fig.5: die andere Stirnansicht sowie
- Fig.6: eine Draufsicht auf einen Längsschlitz des Hilfsgriffes gemäß den Figuren 1 und 2,
- Fig.7: eine Stirnansicht eines abgewandelten Hilfsgriffes, bei welchem ein in radialer und axialer Richtung verlaufender Schlitz keilförmig ausgebildet ist und an den Schlitzwänden zueinander passende Führungen vorgesehen sind, die beim Zusammendrücken des Hilfsgriffes zusammenwirken und ineinandergreifen,
- Fig.8: eine Stirnansicht eines in Längsrichtung zweigeteilten Hilfsgriffes, dessen beide Hälften in ihrem Berührbereich Kupplungsmittel zumindest zum gegenseitigen Festlegen gegen ein gegenseitiges Verschieben der beiden Teile aufweisen,
- Fig.9: eine Stirnansicht eines Hilfsgriffes, der neben einem durch seine Oberfläche reichenden Längsschlitz noch zwei seine Flexibilität erhöhende, in radialer Richtung verlaufende Schlitze oder Ausnehmungen hat, die unterhalb seiner Oberfläche enden,
- Fig.10: einen Längsschnitt durch einen in Gebrauchsstellung befindlichen Hilfsgriff, der eine eine Druckfeder aufweisende, in radialer Richtung wirkende Klemme enthält,
- Fig.11: eine der Fig.10 entsprechende Darstellung eines längsgeschnittenen Hilfsgriffes, der eine um eine quer zu der Längsinnenhöhlung des Hilfsgriffes und quer zu dem Katheter angeordnete, exzentrisch gelagerte Klemmrolle zum lösbaren Festklemmen des Hilfsgriffes an dem Katheter aufweist, die mit einem Teil ihres Umfanges über die Oberseite des Hilfsgriffes zur Durchführung einer Drehbewegung übersteht,
- Fig.12: eine Seitenansicht und
- Fig.13: einen Querschnitt einer abgewandelten Ausführungsform, bei welcher der Hilfsgriff eine in radialer Richtung wirkende Klemme dadurch enthält, daß der Querschnitt der Innenlängshöhlung zwei verschiedene Abmessungen hat, wobei eine kleinere Abmessung kleiner als der Querschnitt des Katheters und die demgegenüber größere Abmessung größer als dieser Querschnitt ist,
- Fig.14: den Querschnitt gemäß Fig.13 beim seitlichen Einführen des Katheters in die Innenlängshöhlung oder Aufstecken des Hilfsgriffes auf den Katheter, wobei eine leichte Spreizung des axial und radial verlaufenden Einführ-Schlitzes angedeutet ist,
- Fig.15: den Querschnitt des Hilfsgriffes gemäß den Fig. 13 und 14 mit in der Innenlängshöhlung aufgrund der Formgebung und Elastizität des Werkstoffes des Hilfsgriffes oder einer Auskleidung selbsttätig eingeklemmtem Katheter,
- Fig.16: den Querschnitt gemäß Fig.15, wobei entgegengesetzte Druckkräfte in Richtung der längeren Querschnittsabmessung der Innenlängshöhlung zu einer Verringerung dieser Abmessung und gleichzeitiger Erweiterung der quer dazu verlaufenden Abmessung führen und die Verklemmung mit dem Katheter dadurch aufgehoben und gelöst ist, so daß eine relative Längsverschiebung zwischen Katheter und Hilfsgriff möglich ist,
- Fig.17: einen Längsschnitt und
- Fig.18: einen Querschnitt eines Hilfsgriffes, dessen Innenlängshöhlung mit einer bis zu dem Einführ-Schlitz reichenden, ihrerseits aber auch geschlitzten Auskleidung aus einer Haftfolie versehen ist, die bei Andruck an einen Katheter eine erhöhte Reibung ergibt, so daß eine geringere Druckkraft zum Festlegen des Hilfsgriffes ausreicht,
- Fig.19: einen Querschnitt und
- Fig.20: einen Längsschnitt einer abgewandelten Ausführungsform, bei welcher der Hilfsgriff zweiteilig dadurch ausgebildet ist, daß eine innere Auskleidung verdrehbar in der Innenlängshöhlung angeordnet ist und dazu an einem Ende formschlüssig und am anderen Ende an einem überstehenden Kopf befestigt ist, der gegenüber dem Hilfsgriff verdrehbar und stirnseitig in unterschiedlichen Drehstellungen verrastbar ist,
- Fig.21: einen Längsschnitt der Innenauskleidung mit einem an einem Ende angeordneten Mehrkant und dem am entgegengesetzten Ende angeordneten Kopf vor der Montage an dem Hilfsgriff,
- Fig.22: einen Längsschnitt des Hilfsgriffes ohne die Innenauskleidung gemäß Fig.21,
- Fig.23: eine Stirnansicht des Hilfsgriffes gemäß Figur 22 mit Blick auf radiale Vertiefungen für die Verrastung oder Aufnahme entsprechender Vorsprünge an der dieser Stirnseite zugewandten Unterseite des Kopfes der Innenauskleidung,
- Fig.24: eine teilweise im Längsschnitt gehaltene Seitenansicht des Hilfsgriffes gemäß den Figuren 20 bis 22 in Ausgangsstellung, in welcher ein Katheter eingelegt bzw. der Hilfsgriff über einen Katheter seitlich aufgesteckt werden kann, sowie
- Fig.25: eine der Fig.24 entsprechende, teilweise im Längsschnitt gehaltene Seitenansicht, nachdem der Kopf der Innenauskleidung relativ zu dem übrigen Hilfsgriff verdreht und dadurch die Innenauskleidung unter Verkleinerung ihres Innenquerschnittes verdrillt ist, um an einem innenliegenden Katheter eine Klemmung zu bewirken, die selbsttätig fixiert, aber durch eine Zugkraft an dem Kopf zum Entriegeln der stirnseitigen Verrastung lösbar ist.

Bei den nachfolgend beschriebenen Ausführungsbeispielen werden von der Funktion her übereinstimmende Teile oder Bereiche mit übereinstimmenden Bezugszahlen versehen, auch wenn sie unterschiedlich gestaltet sind. Dabei gelten Beschreibungen dieser Teile oder Bereiche, die nur im Zusammenhang mit einer oder einzelnen Figuren erwähnt sind, auch für solche Figuren und Ausführungsbeispiele, bei denen sie nicht ausdrücklich erwähnt, aber dennoch anwendbar sind.

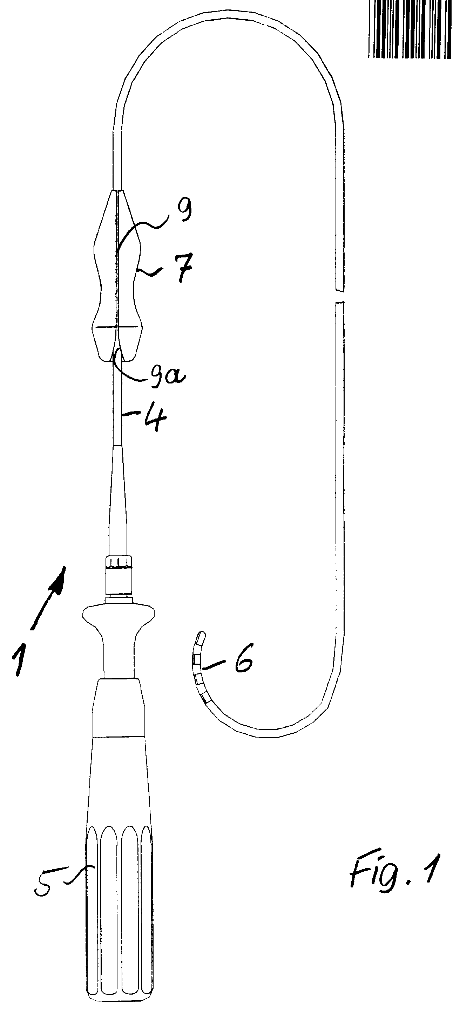

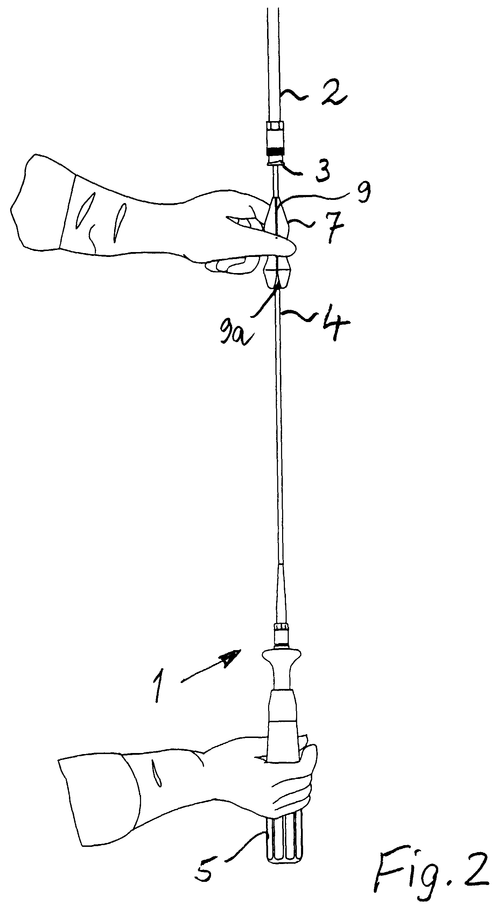

Ein in Fig.1 im ganzen mit 1 bezeichneter Katheter dient gemäß Fig.2 zum Einführen in das Innere eines Körpers beispielsweise eines Menschen, wobei dieser Körper nicht dargestellt ist, aber eine Einführvorrichtung 2. Diese führt beispielsweise in ein Blutgefäß und dichtet den Katheter an seinem Eintritt 3 ab, so daß kein Blut austreten kann.

An dem aus dem Körper und dieser Einführvorrichtung 2 überstehenden Endbereich 4 hat der Katheter 1 einen Handgriff 5, womit Manipulationen der Arbeitsspitze 6 (vgl.Fig.1), insbesondere ihre Positionierung oder auch eine Änderung ihrer Positionierung möglich ist.

In Fig.1 und 2 erkennt man ferner, daß an dem außerhalb des Körpers bleibenden Endbereich 4 des Katheters 1 ein zusätzlicher, relativ zu dem Katheter 1 verschiebbarer, durch eine noch näher zu erläuternde radiale Druckkraft an der Außenseite des Katheters zeitweise und lösbar festlegbarer Hilfsgriff 7 vorgesehen ist. Dadurch ist es möglich, daß ein Benutzer oder Operateur den Katheter 1 nicht nur mit Hilfe des Handgriffes 5 und einem mitunter beträchtlichen Abstand zu dem Eintritt 3 in die Einführvorrichtung 2 und in den Körper des Patienten handhabt und manipuliert, sondern er kann mit der zweiten Hand an diesem Hilfsgriff 7 anfassen, dadurch den Katheter 1 besser führen und zusätzlich manipulieren. Durch die Verschiebbarkeit und Lösbarkeit kann dabei die Angriffsstelle dieses Hilfsgriffes 7 beliebig gewählt und auch während der Behandlung geändert werden.

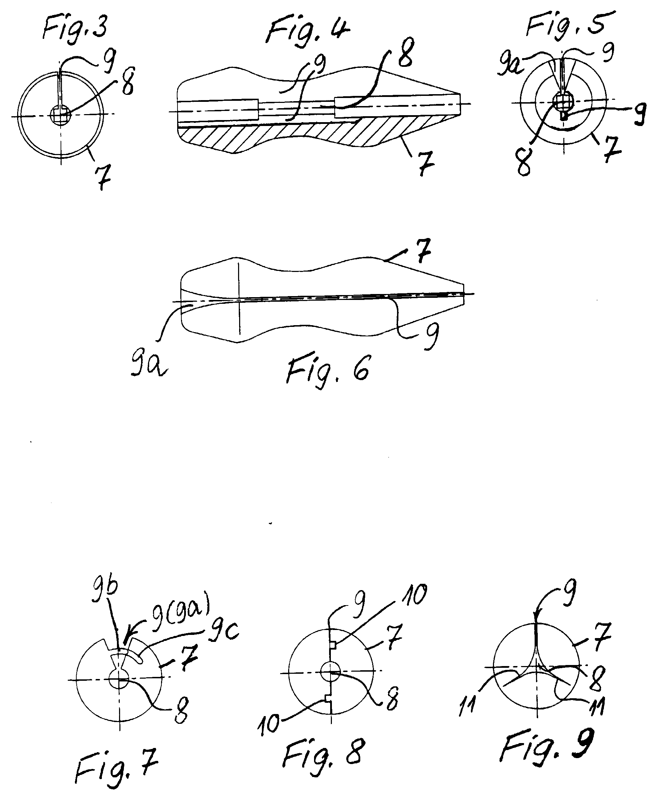

Gemäß den Figuren 3 bis 25 hat der Hilfsgriff 7 eine den Katheter 1 bzw. dessen Endbereich 4 aufnehmende Innenlängshöhlung 8, so daß eine gute Kraftübertragung möglich ist. Im Ausführungsbeispiel gemäß den Figuren 1 bis 9 oder 17 und 18 ist dabei der Hilfsgriff 7 bzw. seine Innenlängshöhlung 8 zumindest bereichsweise derart zusammendrückbar, daß dieser Druck sich auf den Katheter 4 fortsetzt und Reibschluß zwischen ihm und dem Hilfsgriff 7 herstellt. Somit ist in diesem Ausführungsbeispiel der Hilfsgriff 7 durch Druck auf seine Außenseite lösbar an dem Katheter 1 festklemmbar, wobei diese Druckkraft von der haltenden Hand gleichzeitig durch die Haltekraft bereits aufgebracht werden kann. Dabei kann diese Druckkraft in jeder beliebigen Querrichtung, also, wie in Fig.2 sichtbar, auch teilweise in Richtung der radialen Erstreckung eines noch zu erläuternden axialen Schlitzes 9 erfolgen, wenn der Hilfsgriff 7 aus entsprechend weichem Werkstoff wie weichem Gummi, Kunstgummi oder Kunststoff besteht.

Gemäß Fig.3 ist dabei die Lochung oder Innenlängshöhlung 8 zumindest in einem in Fig.4 erkennbaren mittleren engeren Bereich profiliert und hat dabei einen viel- oder viereckigen, in diesem Ausführungsbeispiel quadratischen Innenquerschnitt. Dies führt in vorteilhafter Weise zu einer nur linien- oder gegebenenfalls nur punktförmigen Berührung zwischen der Innenlängshöhlung 8 und dem von ihr in Gebrauchsstellung umschlossenen Katheter 1, so daß die gewünschte Verschiebbarkeit entsprechend leichtgängig ist, dennoch aber beim Festhalten und Zusammendrücken eine große Haltekraft entsteht.

Der schon erwähnte axial und radial verlaufende Schlitz 9, der von der Innenlängshöhlung 8 ausgeht und gemäß Figur 3 bis 25 die Oberfläche des Hilfsgriffes 7 durchsetzt, ermöglicht eine Aufweitung des Hilfsgriffes 7 entlang diesem Schlitz 9, um ihn in Querrichtung auf den Katheter 1 aufzustecken oder umgekehrt von ihm zu entfernen. Somit kann der Hilfsgriff 7 auch dann an dem Katheter nachträglich angebracht werden, wenn er gemäß Fig.2 schon verlegt ist. Ferner kann der Hilfsgriff 7 nach der Benutzung entfernt werden, so daß er selbst bei nur einmaligem Gebrauch des Katheters 1 mehrfach Verwendung finden kann. Außerdem können die Fertigung des Katheters 1 und des Hilfsgriffes 7 völlig unabhängig voneinander erfolgen, weil der Hilfsgriff 7 zu jeder beliebigen Zeit in Querrichtung auf den Katheter 1 aufgesteckt werden kann unabhängig von Form und Größe der beidseits sich erstreckenden Katheterteile oder Anbauten. Ein Einfädeln des Katheters in den Hilfsgriff 7 wird durch diese Schlitzung vermieden.

Dabei ist in Fig.4 dargestellt, daß dieser Schlitz 9 von außen her in radialer Richtung bis unter die Mitte des Hilfsgriffes 7 und bis unter die Innenlängshöhlung 8 reicht, zumindest über einen Teil der axialen Länge des Hilfsgriffes 7. Dadurch wird der Hilfsgriff weiter "geschwächt", kann also umso leichter im Bereich des Schlitzes geöffnet werden, um den Hilfsgriff über einen Katheter 1 aufzustecken, und außerdem sind die Rückstellkräfte innerhalb des Hilfsgriffes 7 verringert, so daß auch die Verschiebbarkeit auf dem Katheter entsprechend leichtgängig ist, bis der Hilfsgriff durch den Benutzer angedrückt wird. Der Schlitz 9 schwächt also die Festigkeit des Hilfsgriffes, so daß er einerseits leichtgängig verschiebbar ist, andererseits aber eine geringere Druckkraft auf seine Außenseite schon ausreicht, um eine genügende Reibkraft im Bereich der zum Teil verengten (Fig.4) Innenlängshöhlung 8 zu bewirken.

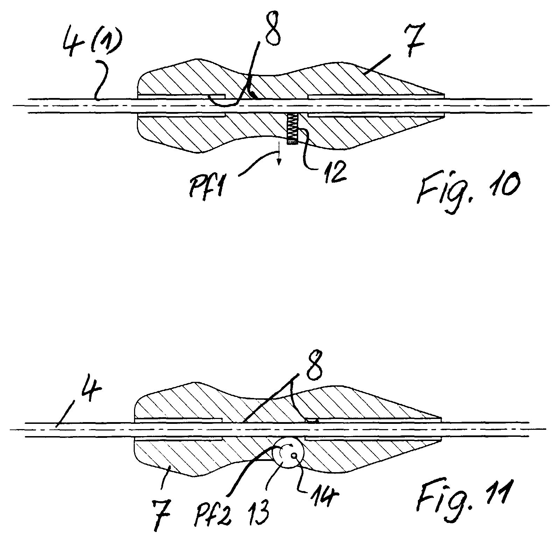

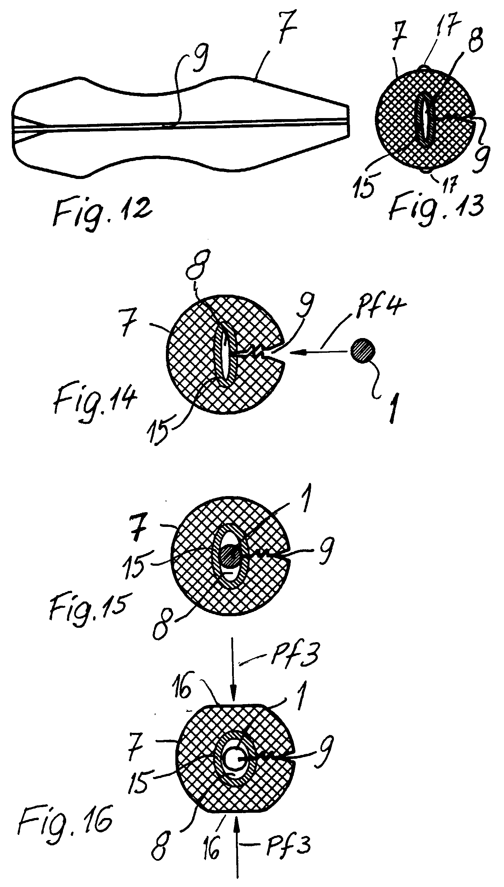

Es ergibt sich also ein sehr einfach aufgebauter Hilfsgriff 7, der schnell und einfach an beliebige Katheter anmontiert werden kann, wobei seine zeitweise Fixierung durch den Benutzer selbst und durch dessen Haltekraft erfolgt. Vor allem bei Manipulationen mit Hilfe des Hilfsgriffes 7 erhöht sich dabei die Haltekraft und damit die Klemmkraft praktisch von selbst.
Die Form des Schlitzes 9 kann dabei unterschiedlich sein, wie es beispielsweise in Figur 7 bis 9, 13 bis 16, 18 oder 19 angedeutet ist. Aber auch im Verlauf des Schlitzes selbst kann gemäß Fig.6 oder 12 eine Formänderung vorgesehen sein, die das seitliche Aufstecken aufgrund einer trichterförmigen Erweiterung 9a an wenigstens einem Ende des Hilfsgriffes 7 oder aufgrund einer über die gesamte Schlitzlänge verlaufenden trichterförmigen Querschnittsgestaltung (Fig.13 bis 19) erleichtert.

Fig.7 zeigt eine Anordnung, bei welcher sich der Schlitz 9 insgesamt nach außen erweitert, wobei an den Schlitzwänden zueinanderpassende und beim Zusammendrücken ineinandergreifende Führungsmittel in Form eines Vorsprunges 9b auf der einen Seite und einer dazu passenden Aussparung 9c auf der anderen Seite vorgesehen sind.

Fig.8 zeigt eine Ausführungsform, bei welcher der Hilfsgriff 7 durch axial und radial verlaufende Schlitze in zwei jeweils einen Anteil der Innenlängshöhlung 8 aufweisende Teile, in diesem Falle Hälften, zerlegbar ist, wobei Verbindungsmittel 10 zum Zusammenhalten in Gebrauchsstellung und zum gegenseitigen Kuppeln der beiden Teile oder Hälften vorgesehen sind. Dabei genügt es, wenn diese Verbindungsmittel 10 ein gegenseitiges Verschieben der beiden Teile verhindern, während das Zusammenhalten selbst evtl. sogar nur durch die haltende Hand erfolgen könnte, aber auch durch Reibschluß oder Clipse oder dergleichen unterstützt sein könnte.

Fig.9 zeigt eine Ausführungsform, bei welcher zusätzlich zu dem die Oberfläche des Hilfsgriffes 7 durchsetzenden Schlitz 9 zwei weitere axial und radial zu der Innenlängshöhlung 8 verlaufende Schlitze 11 vorgesehen sind, die das Aufweiten des Schlitzes 9 zum Aufstecken auf den Katheter 1 erleichtern können. Diese zusätzlichen Schlitze 11 enden dabei unterhalb der Oberfläche des Hilfsgriffes 7. Gleichzeitig vermindern sie die Rückstellkraft beim Aufweiten des offenen Schlitzes 9, so daß das nachträgliche Anbringen an einem Katheter 1 entsprechend erleichtert ist.

In den Figuren 10, 11, 12 bis 16 und 19 bis 25 sind Beispiele von Hilfsgriffen 7 dargestellt, die eine nach seiner Anbringung wirksame Klemme enthalten, welche Klemme durch eine spezielle Lösebewegung öffenbar oder lösbar ist.

Der Hilfsgriff 7 gemäß Fig.10 enthält dabei wenigstens eine in radialer Richtung wirksame Druckfeder 12 als Klemme, die in einer klemmenden Druckposition lösbar gehalten und durch eine Ausrastbewegung entspannbar ist. Durch einen Druck entgegen dem Pfeil Pf1 kann die Druckfeder 12 gespannt und dadurch die Klemmwirkung erzeugt und durch eine weitere Druckbewegung in der gleichen Richtung wieder gelöst werden, so daß sie sich in Richtung des Pfeiles Pf1 entspannen kann.

Fig.11 zeigt eine Anordnung, bei welcher als Klemme eine exzentrisch angeordnete Rolle 13 innerhalb des Hilfsgriffes 7 drehbar gelagert ist, deren exzentrische Achse 14 quer zu der Längsmittelhöhlung 8 und quer zu dem Katheter 1 orientiert ist und die zum Verklemmen in Richtung des Pfeiles Pf2 und zum Lösen entgegen dieser Klemmstellung verdrehbar ist. Beim Verdrehen an dem über die Oberfläche des Hilfsgriffes 7 vorstehenden Umfangsteil der Rolle 13 gelangt ein Bereich größerer Exzentrizität zu dem Katheter 1 und kann diesen klemmend beaufschlagen. Bei der umgekehrten Verdrehung wird dieser Bereich größerer Exzentrizität von dem Katheter 1 wieder wegbewegt, so daß der Katheter 1 freigegeben wird.

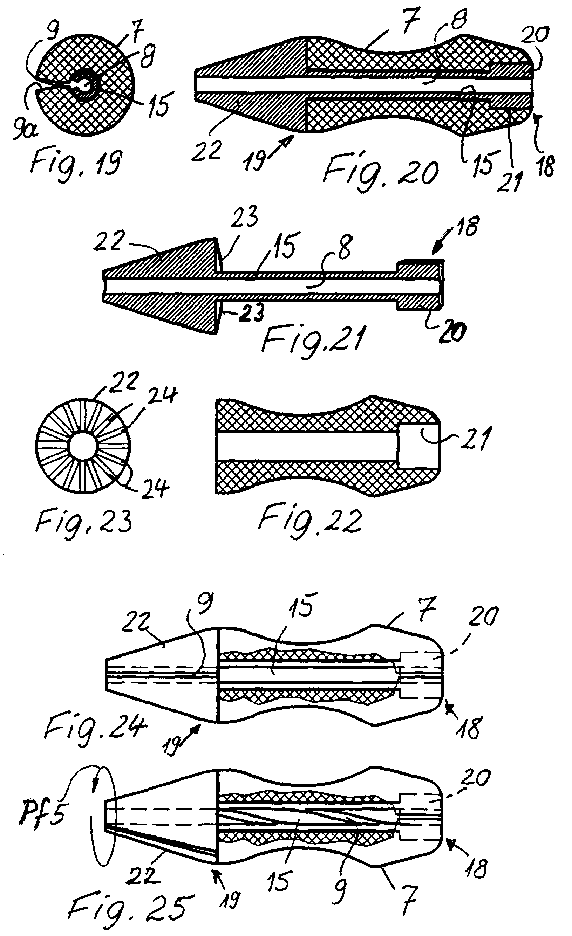

Die Ausführungsformen gemäß Figur 12 bis 16 einerseits und auch gemäß Figur 19 bis 25 zeigen jeweils Beispiele von Hilfsgriffen 7, bei denen die darin enthaltene Klemme durch die Form der Innenlängshöhlung 8 und/oder des Hilfsgriffes 7 selbst gebildet ist, das heißt die Innenlängshöhlung 8 und in gewissem Umfange der Hilfsgriff 7 sind dabei selbst als verformbare oder lösbare Klemme ausgebildet.

Gemäß Fig.12 bis 16 ist vorgesehen, daß die Innenlängshöhlung 8 in Ausgangslage gemäß Fig.13 und 14 einen von dem Katheterquerschnitt abweichenden Hohlquerschnitt aufweist, wobei im Ausführungsbeispiel ein etwa kreisförmiger Katheterquerschnitt dargestellt ist, während der Hohlquerschnitt der Innenlängshöhlung länglich oder oval oder ellipsenförmig oder etwa daran angenähert gestaltet ist. Dabei ist der Umfang des Hohlquerschnittes der Innenlängshöhlung 8 zumindest bereichsweise größer als der Umfang des Katheters 1, wie man es besonders gut in Fig.15 erkennt. Die Innenwand der Innenlängshöhlung 8 oder einer noch zu beschreibenden Auskleidung 15 dieser Innenlängshöhlung 8 liegt dabei gemäß Fig.15 in Gebrauchsstellung an einem Teil des Umfanges des Katheters 1 an und hat in Nachbarbereichen einen Abstand davon. Konkret hat die Innenlängshöhlung 8 einen länglichen, in Richtung seiner größten Ausdehnung zusammendrückbaren Querschnitt, während die kleinere Abmessung dieses Querschnittes vor dem Einsetzen des Katheters 1 gemäß Fig.14 kleiner als die entsprechende Querschnittsabmessung des Katheters 1 ist. Es leuchtet ein, daß aufgrund dieser geometrischen Verhältnisse und der Eigenelastizität des Hilfsgriffes 7 und/oder der Auskleidung 15 der Katheter 1 nach dem Zusammenfügen mit dem Hilfsgriff 7 in der Innenlängshöhlung 8 selbsttätig eingeklemmt wird bzw. seinerseits umgekehrt den Innenquerschnitt der Innenlängshöhlung 8 etwas aufweitet.

Wird nun in Richtung der größeren Querschnittsabmessung der Innenlängshöhlung 8 gemäß den beiden einander entgegengesetzten Pfeilen Pf3 (Fig.16) eine Druckkraft ausgeübt und dadurch diese längere Querschnittsabmessung verkürzt, vergrößert sich automatisch die etwa rechtwinklig dazu angeordnete kleinere Querschnittsabmessung und gibt den Katheter, wie in Fig.16 dargestellt, am gesamten Umfang frei, das heißt unter einer solchen Druckkraft gemäß den beiden Pfeilen Pf3 kann die Klemmwirkung zwischen Hilfsgriff 7 und Katheter 1 aufgehoben oder beseitigt werden, so daß der Hilfsgriff 7 und der Katheter 1 relativ zueinander leicht verschoben werden können.

Es wird also in diesem Falle umgekehrt wie bei den vorangegangenen Ausführungsbeispielen von dem Benutzer der Druck auf den Hilfsgriff 7 dazu benutzt, um die lösbare Verbindung, eine Klemmverbindung, zu dem Katheter 1 zu lösen, während in den Ausführungsbeispielen gemäß Fig.1 bis 9 die Druckkraft dazu dient, die Klemmwirkung zwischen Hilfsgriff 7 und Katheter 1 zu erzeugen. Beiden Lösungen ist jedoch gemeinsam, daß der Hilfsgriff 7 durch eine radiale Druckkraft an der Außenseite des Katheters 1 lösbar festlegbar ist.

Dabei wird auch gleichzeitig deutlich, daß der Querschnitt des Schlitzes 9, der zum seitlichen Einführen des Katheters 1 dient, wie es in Fig.14 durch den Pfeil Pf4 angedeutet ist, quer oder rechtwinklig zur größeren Querschnittsabmessung der im Querschnitt länglichen Innenlängshöhlung 8 angeordnet ist. Wird die in Fig.16 durch die Pfeile Pf3 angedeutete Druckkraft ausgeübt, um die Verschiebung des Hilfsgriffes 7 gegenüber dem Katheter 1 beziehungsweise eine Verschiebung des Katheters 1 gegenüber dem Hilfsgriff 7 zu erlauben, wird also dieser Schlitz 9 zusätzlich zugedrückt, so daß ein unbeabsichtigtes Lösen des Hilfsgriffes 7 vom Katheter 1 bei dieser Manipulation praktisch ausgeschlossen wird.

Zusätzlich ist der Schlitz 9 im Inneren noch profiliert, so daß in Schließstellung eine Verzahnung gegeben ist, die ein ungewolltes Trennen des Hilfsgriffes 7 von dem Katheter 1 weiter erschwert, also die Sicherheit der an sich lösbaren Verbindung verbessert.

In Fig.16 ist noch angedeutet, daß der Hilfsgriff 7 eine Außenform oder -profilierung in Form von Abplattungen 16 haben kann, wodurch die Druckrichtung zum Zusammendrücken des Hilfsgriffes 7 für den Benutzer vorgegeben bzw. gut erkennbar gemacht wird, so daß das Lösen des als Klemme ausgebildeten und durch Eigenspannung an der Katheteraußenseite festliegenden Hilfsgriffes 7 erleichtert wird. Statt dieser Profilierung können gemäß Fig.13 auch fühlbare Markierungen 17 in Form von Noppen oder dergleichen Vorsprüngen vorgesehen sein. Ferner können diese Formgebungen oder fühlbaren Markierungen auch bei den Ausführungsbeispielen gemäß Fig.3 bis 9 vorgesehen sein, wo das Aufbringen der Druckkraft zur Fixierung und nicht zum Lösen des Hilfsgriffes 7 dient. Außerdem erleichtert eine solche Profilierung oder Formgebung die Handhabung des Hilfsgriffes 7 und verbessert auch die Möglichkeit seiner Verdrehung zusammen mit dem Katheter 1 oder relativ dazu.

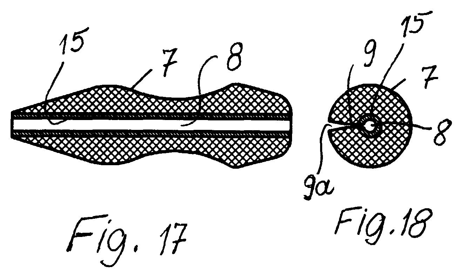

In Fig.17 und 18 ist eine abgewandelte Ausführungsform des Hilfsgriffes 7 dargestellt, der für eine zeitweilige Fixierung an einem Katheter 1 analog den Ausführungsbeispielen gemäß Fig.3 bis 9 an der Außenseite zusammengedrückt wird, während bei Aufhebung einer solchen zeitweiligen Druckkraft die Verschiebung ermöglicht wird. Sowohl in diesem Ausführungsbeispiel gemäß Fig.17 und 18 als aber auch in dem schon beschriebenen Ausführungsbeispiel gemäß Fig.13 bis 16 ist der Hilfsgriff 7 in seiner Innenlängshöhlung 8 zumindest bereichsweise mit einer unter Druck gleithemmenden oder rutschfesten Auskleidung 15 versehen. Dadurch wird die Funktion des Hilfsgriffes auch unter ungünstigen Bedingungen, zum Beispiel an einem feuchten oder nassen Katheter 1 ermöglicht und in der Regel wird die erforderliche Druckraft verringert, um eine zeitweilige Fixierung zu erreichen. Dies ist besonders vorteilhaft, wenn die elastischen Rückstellkräfte der Innenauskleidung 15 bzw. des Hilfsgriffes 7 gemäß Fig.12 bis 16 zur Fixierung vorgesehen sind.

Die Auskleidung 15 kann eine Folie mit an ihrer Oberfläche überstehenden Partikeln, zum Beispiel eine Schmirgelfolie, oder eine Folie mit glatter Oberfläche und hohem Reibungskoeffizienten, insbesondere aus Polyurethan, sein. Dabei ist diese Auskleidung 15 natürlich an derselben Stelle wie der Hilfsgriff 7 mit einem Längsschlitz für das seitliche Einführen des Katheters 1 versehen, der praktisch zu dem Schlitz 9 gehört bzw. diesen fortsetzt.

Fig.19 bis 25 zeigt ebenfalls ein Ausführungsbeispiel des Hilfsgriffes 7 mit einer Auskleidung 15 der Innenlängshöhlung, wobei nun diese Auskleidung 15 selbst mehr oder weniger unabhängig von dem Hilfsgriff 7 zur zeitweiligen Fixierung des Hilfsgriffes 7 an einer gewünschten Stelle des Katheters 1 benutzt wird.

Dies geschieht dabei dadurch, daß die lichte Weite des Innenquerschnittes der Innenlängshöhlung 8 und der Auskleidung 15 durch Verdrillen verringert wird und in einer so verformten verdrillten Position lösbar festgelegt werden kann. Gemäß Fig.20 ist dabei die Auskleidung 15 der Innenlängshöhlung 8, welche Auskleidung 15 also praktisch dann selbst die Innenlängshöhlung 8 zur Aufnahme des Katheters 1 enthält, in einem ersten Endbereich 18 in noch zu beschreibender Weise drehfest mit dem Hilfsgriff 7 und an dem anderen zweiten Endbereich 19 verdrehbar und lösbar festlegbar ausgebildet.

Fig.21 zeigt die Gestaltung dieser Auskleidung 15, die an dem Endbereich 18 eine vieleckige, zum Beispiel vierkantförmige Verdickung 20 aufweist, die in ein in Fig.22 erkennbares, entsprechend geformtes Gegenprofil 21 des Hilfsgriffes 7 paßt und in Gebrauchsstellung gemäß Fig.20 darin eingefügt und eingesetzt ist.

Am entgegengesetzten Ende ist an der Auskleidung 15 ein in Gebrauchsstellung gemäß Fig.20, 24 und 25 gegenüber dem Hilfsgriff 7 axial vorstehender Kopf 22 zum lösbaren Verrasten oder Verriegeln gegenüber dem Hilfsgriff 7 vorzugsweise in unterschiedlichen Drehpositionen vorgesehen. Dabei ist die Auskleidung 15 zumindest zwischen der Verdickung 20 und dem Kopf 22 auch in axialer Richtung elastisch.

In Fig.21 erkennt man an der Unterseite des Kopfes 22 in radialer Richtung von innen nach außen verlaufende, gegenüber der Unterseite des Kopfes 22 axial etwas vorstehende Rippen 23, während in Fig.23 die Stirnseite des Hilfsgriffes 7 auf der Seite dieses Hilfsgriffes 7 dargestellt ist, an welcher die Unterseite des Kopfes 22 in Gebrauchsstellung zur Anlage kommt. Diese Stirnseite hat radial verlaufende Nuten oder dergleichen Vertiefungen 24, in welche die Rippen 23 passen. Auch eine umgekehrte Anordnung ist möglich.

Die Ausgangslage der Anordnung dieses Ausführungsbeispieles des Hilfsgriffes ist in den Figuren 20 und 24 erkennbar. In dieser Lage ist der Hilfsgriff 7 gegenüber einem in diesem Falle nicht dargestellten, innerhalb der Innenlängshöhlung 8 verlaufenden Katheter 1 leicht verschieblich.

Soll der Hilfsgriff 7 für eine gewisse Zeit an einer bestimmten Stelle des Katheters 1 lösbar festgelegt oder festgeklemmt werden, wird der Kopf 22 relativ zu dem übrigen Hilfsgriff 7 etwa in Richtung des Pfeiles Pf5 in Fig.25 verdreht, wodurch die Auskleidung 15 in der in Figur 25 erkennbaren Weise verdrillt wird. Man erkennt deutlich die Schraubenlinienform des Einführschlitzes 9 der Auskleidung 15, die durch eine solche Verdrillung entsteht.

Durch diese Verdrillung ergibt sich eine Zugkraft im Inneren der Auskleidung 15 und vor allem auch eine Verkleinerung von deren Innenquerschnitt, so daß ein dort verlaufender Katheter klemmend umfaßt wird, während außerdem der Kopf 22 nach dem Loslassen durch den Benutzer mit seinen Rippen 23 in entsprechende Vertiefungen 24 gezogen und dadurch die eingestellte Lage fixiert werden.

Soll der Hilfsgriff 7 wieder gelöst werden, übt der Benutzer an dem Kopf 22 einen leichten Zug aus und macht die Verdrillung durch Rückdrehen rückgängig.

Selbstverständlich kann die Verdrillung auch durch eine dem Pfeil Pf5 entgegengesetzte Drehung des Kopfes 22 relativ zu dem Hilfsgriff 7 erreicht werden.

Die Montage der Auskleidung an dem Hilfsgriff 7 ist sehr einfach, weil dieser den seitlichen Einführ-Schlitz 9 aufweist, durch den die elastische Innenauskleidung 15 in gleicher Weise seitlich eingeführt werden kann, wie dies später auch mit dem Katheter 1 geschieht. Da die Auskleidung 15 elastisch ist, kann gleichzeitig durch eine Zugkraft der Außenquerschnitt der Auskleidung 15 vermindert und die Verdickung 20 in axialer Richtung außerhalb des Bereiches des Hilfsgriffes gebracht werden, so daß sie dann nach dem Einführen der Auskleidung in das Innere des Hilfsgriffes 7 durch Entspannen in axialer Richtung in die profilierte Aussparung 21 eintreten kann. Gegebenenfalls können die Abmessungen dabei so gewählt werden, daß auch in der in Fig.20 und 24 dargestellten Ruhelage ein leichter axialer Zug zwischen Verdickung 20 und Kopf 22 besteht, um die Anordnung zu stabilisieren.

Der Hilfsgriff 7 kann an einem außerhalb eines Körpers bleibenden Bereich 4 eines Katheters 1 angeklemmt sein oder angeklemmt werden und ist gegenüber dem Katheter 1 in dessen Erstreckungsrichtung verschiebbar, aber auch insbesondere durch die Haltekraft des Benutzers einklemmen oder durch Elastizitäts- oder Rückstellkräfte festklemmbar, so daß der Benutzer den Katheter 1 nicht nur mit dessen Handgriff 5, sondern zusätzlich mit diesem Hilfsgriff 7 bewegen, manipulieren oder wenigstens bequem halten kann. Da der Hilfsgriff 7 lösbar befestigt ist, kann er gegebenenfalls auch mehrfach wiederverwendet werden.

## Patentansprüche

1. Katheter (1) zum Einführen in das Innere des Körpers eines Lebewesens, insbesondere eines Menschen und vorzugsweise in das Gefäßsystem, welcher Katheter einen in Gebrauchsstellung aus dem Körper überstehenden Endbereich (4) mit wenigstens einem Handgriff oder Manipulator oder dergleichen Angreifstelle zur Durchführung von Manipulationen aufweist, **dadurch gekennzeichnet,** daß an dem außerhalb des Körpers bleibenden Endbereich (4) des Katheters (1) ein zusätzlicher relativ zu ihm verschiebbarer, durch eine radiale Druckkraft an der Außenseite des Katheters lösbar festlegbarer Hilfsgriff (7) vorgesehen ist.

2. Katheter nach Anspruch 1, dadurch gekennzeichnet, daß der Hilfsgriff (7) eine den Katheter (1) aufnehmende Innenlängshöhlung (8) aufweist und mindestens bereichsweise derart zusammendrückbar ist, daß er durch Druck auf seine Außenseite lösbar festklemmbar ist.

3. Katheter nach Anspruch 1 oder 2, dadurch gekennzeichnet, daß die Innenlängshöhlung (8) zumindest in einem mittleren oder engeren Bereich profiliert ist und insbesondere einen viel- oder viereckigen oder quadratischen Innenquerschnitt hat.

4. Katheter nach einem der Ansprüche 1 bis 3, dadurch gekennzeichnet, daß der Hilfsgriff (7) aus weichem Gummi, Kunstgummi oder Kunststoff besteht und vom Benutzer durch den Haltedruck mit der Katheteraußenseite verklemmbar ist.

5. Katheter nach einem der Ansprüche 1 bis 4, dadurch gekennzeichnet, daß der Hilfsgriff (7) - ausgehend von seiner Innenlängshöhlung (8) - wenigstens einen axial und radial verlaufenden Schlitz (9) aufweist, der seine Oberfläche

6. Katheter nach einem der Ansprüche 1 bis 5, dadurch gekennzeichnet, daß der Hilfsgriff (7) durch axiale und radiale Schlitze (9) in wenigstens zwei jeweils einen Anteil der Innenlängshöhlung (8) aufweisende Teile zerlegbar ist und Verbindungsmittel (10) zum Zusammenhalten in Gebrauchsstellung, insbesondere zum gegenseitigen Kuppeln der beiden Teile oder Hälften, aufweist.

7. Katheter nach einem der Ansprüche 1 bis 6, dadurch gekennzeichnet, daß von der Innenlängshöhlung (8) aus wenigstens ein weiterer, in radialer und axialer Richtung verlaufender Schlitz (11) oder dergleichen Aussparung vorgesehen ist, welche unterhalb der Oberfläche des Hilfsgriffes (7) endet.

8. Katheter nach einem der Ansprüche 1 bis 7, dadurch gekennzeichnet, daß der Hilfsgriff (7) eine nach seiner Anbringung wirksame Klemme enthält, die durch eine Lösebewegung öffenbar oder lösbar ist.

9. Kathether nach einem der Ansprüche 1 bis 8, dadurch gekennzeichnet, daß als Klemme eine exzentrisch angeordnete Rolle (13) innerhalb des Hilfsgriffes (7) gelagert ist, deren Achse (14) quer zu der Längsmittelhöhlung (8) des Hilfsgriffes (7) orientiert ist und die zum Lösen entgegen ihrer Klemmstellung verdrehbar ist, und/oder wenigstens eine in radialer Richtung wirksame Druckfeder (12) vorgesehen ist, die in einer klemmenden Druckposition lösbar gehalten und durch eine Ausrastbewegung entspannbar ist.

10. Katheter nach einem der Ansprüche 1 bis 9, dadurch gekennzeichnet, daß der die Oberfläche des Hilfsgriffes (7) durchsetzende Schlitz (9) an zumindest einem Ende des Hilfsgriffes (7) eine insbesondere etwa trichterförmige Erweiterung (9a) hat.

11. Katheter nach einem der Ansprüche 1 bis 10, dadurch gekennzeichnet, daß die Innenlängshöhlung (8) und/oder der Hilfsgriff (7) selbst als verformbare oder lösbare Klemme ausgebildet sind.

12. Katheter nach einem der Ansprüche 1 bis 11, dadurch gekennzeichnet, daß die Innenlängshöhlung (8) in Ausgangslage einen vom Katheterquerschnitt abweichenden Hohlquerschnitt aufweist, dessen Umfang zumindest bereichsweise größer als der Umfang des Katheters ist und daß die Innenwand der Innenlängshöhlung (8) oder der Auskleidung (15) in Gebrauchsstellung an einem Teil des Umfanges des Katheters (1) anliegt und in Nachbarbereichen einen Abstand davon hat.

13. Katheter nach Anspruch 11 oder 12, dadurch gekennzeichnet, daß die Innenlängshöhlung (8) einen länglichen und in Richtung seiner größten Ausdehnung zusammendrückbaren Querschnitt hat und daß die kleinere Abmessung dieses Querschnittes vor dem Einsetzen des Katheters (1) kleiner als die entsprechende Querschnittsabmessung des Katheters (1) ist.

14. Katheter nach einem der Ansprüche 11 bis 13, dadurch gekennzeichnet, daß der Querschnitt des Schlitzes (9) quer oder rechtwinklig zur größeren Querschnittsabmessung der im Querschnitt länglichen Innenlängshöhlung (8) angeordnet ist.

15. Katheter nach einem der Ansprüche 1 bis 14, dadurch gekennzeichnet, daß der Hilfsgriff (7) eine Außenform oder -profilierung und/oder wenigstens eine fühlbare Markierung (17) für die Druckrichtung zum Zusammendrücken des Hilfsgriffes (7) bei seiner Fixierung an der Katheteraußenseite oder zum Lösen des als Klemme ausgebildeten und durch Eigenspannung an der Katheteraußenseite festliegenden Hilfsgriffes (7) hat.

16. Katheter nach einem der Ansprüche 1 bis 15, dadurch gekennzeichnet, daß der Hilfsgriff (7) in seiner Innenlängshöhlung (8) zumindest bereichsweise mit einer unter Druck gleithemmenden oder rutschfesten Auskleidung (15) versehen ist.

17. Katheter nach Anspruch 16, dadurch gekennzeichnet, daß die Auskleidung (15) eine Folie mit an ihrer Oberfläche überstehenden Partikeln, zum Beispiel eine Schmirgelfolie, oder eine Folie mit glatter Oberfläche und hohem Reibkoeffizienten, insbesondere aus Polyurethan, ist.

18. Katheter nach einem der Ansprüche 1 bis 17, dadurch gekennzeichnet, daß die lichte Weite des Innenquerschnittes der Innenlängshöhlung (8) oder der Auskleidung (15) zumindest bereichsweise durch Längszug und/oder durch Verdrillen verringerbar und in so verformter Position lösbar festlegbar ist.

19. Katheter nach einem der Ansprüche 1 bis 18, dadurch gekennzeichnet, daß die Auskleidung (15) der Innenlängshöhlung (8) in einem Endbereich (18) drehfest mit dem Hilfsgriff (7) und an dem anderen Endbereich (19) verdrehbar und lösbar festlegbar ausgebildet ist und vorzugsweise einen gegenüber dem Hilfsgriff (7) axial vorstehenden Kopf (22) zum Verrasten gegenüber dem Hilfsgriff (7) in unterschiedlichen Drehpositionen hat, wobei die Auskleidung (15) und/oder der Hilfsgriff (7) in axialer Richtung elastisch sind.
